## Europäisches Patentamt

(19) **European Patent Office**

Office européen des brevets

(11) Publication number: **0 101 677**
**B1**

(12) **EUROPEAN PATENT SPECIFICATION**

(45) Date of publication of patent specification: **09.07.86**

(51) Int. Cl.⁴: **C 07 K 7/00, A 61 K 37/00**

(21) Application number: **82900601.4**

(22) Date of filing: **04.01.82**

(86) International application number:
**PCT/US82/00004**

(87) International publication number:
**WO 83/02447 21.07.83 Gazette 83/17**

(54) **ANTIGENIC LINEAR PEPTIDE COMPOUNDS.**

(43) Date of publication of application:
**07.03.84 Bulletin 84/10**

(45) Publication of the grant of the patent:
**09.07.86 Bulletin 86/28**

(84) Designated Contracting States:
**AT DE FR GB LU NL SE**

(56) References cited:
**US-A-4 310 456**

**Biochemical And Biophysical Research Communication, Vol. 74, issued 1977, pages 1066-1070, Wheat et al**

**The Journal of Biological Chemistry, Vol. 254, issued 1979, pages 7621-7623, Musick, et al**

**The Journal of Biological Chemistry, Vol. 251, issued 1976, pages 2044-2048, Chaiken, et al**

**Journal of Molecular Biology, Vol. 104, issued 1977, pages, 659-668, Musick, et al**

(73) Proprietor: **NORTHWESTERN UNIVERSITY Rebecca Crown Center Evanston Illinois 60201 (US)**

(72) Inventor: **GOLDBERG, Erwin 2732 Hampton Parkway Evanston, IL 60201 (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath Maximilianstrasse 58 D-8000 München 22 (DE)**

Courier Press, Leamington Spa, England.

**0 101 677**

## Description

**Background and prior art**

Mammalian spermatozoa have been known to be antigenic for many years. More recently, it has been demonstrated that mammalian sperm contain an antigenic enzyme, which is known as the $C_4$ isozyme of lactate dehydrogenase (LDH-X, LDH-$C_4$). LDH-$C_4$ has been isolated in pure crystalline form from mouse testes. Goldberg (1972) *J. Biol. Chem.* 247:2044—2048. The enzyme has a molecular weight of 140,000 and is composed of four identical C sub-units. The amino acid sequence and three-dimensional structure of LDH-$C_4$ has been studied and partially determined by a number of investigators. See Musick et al (1976) *J. Mol. Biol.* 104:659—668; and Wheat et al (1977) *Biochem & Biophys. Res. Comm.,* 74, No. 3:1066—1077. Wheat et al determined the sequence of the essential thiol peptide from amino acid 159 to 171, and found this to be nearly identical to essential thiol peptides from other vertebrate LDH isozymes.

In 1974, Dr. Erwin Goldberg reviewed the effects of immunication with LDH-X (LDH-$C_4$) on fertility, and advanced the possibility that "by using a defined macromolecular constituent of sperm it becomes possible to elucidate its primary structure in terms of amino acid sequence, to map specifically the antigenic determinant(s) responsible for inducing infertility, and then to construct synthetic peptides containing these determinants. Possessing the capability for synthesizing a molecule with such properties, makes the immunological approach to fertility control feasible". Karolinska Symposia on Research Methods in Reproductive Endocrinolgy, 7th Symposia: Immunological Approaches to Fertility Control, Geneva, 1974 202—222. However, such synthetic antigenic peptides remained a goal and not achievement, although their theoretical desirability has been recognized. In 1979, Dr. Erwin Goldberg summarized the state of the art as follows:

"In conclusion, and on a practical basis, immunotherapy for birth control requires more than effectiveness, specificity, reversibility, and absence of systemic side reaction. Rather large amounts of the antigen must be available in unequivocally pure form. This condition probably cannot be met by a natural product enzyme antigen from sperm or testes. Rather, contraceptive technology requires a synthesizable peptide fragment retaining antigenicity and provoking a response which impairs fertility. Completion of the structural analysis of LDH-$C_4$ should allow mapping of antigenic determinants and synthesis of such peptides for use in a new contraceptive technology". *"Recent advances in Reproduction and Regulation of Fertility",* G. P. Talwar, editor, Elsevier/North Holland Biomedical Press (1979)

**Summary of invention**

It has now been discovered that antigenic peptides can be prepared by synthesizing a linear sequence of 9 to 14 amino acids including the sequence: arginine-methionine-valine-serine-glycine-glutamine-threonine-arginine-leucine. All of the amino acids used to prepare these peptide compounds are in their L-form with the exception of glycine.

The arginine is at the N-terminal and the leucine is at the C-terminal, or in the C-terminal end portion. Although not known with certainty, it is believed that the foregoing sequence of nine amino acids corresponds to amino acids 101 to 109 of LDH-$C_4$. This is contrary to a recently published tentative sequence. Musick et al (1979) *J. Biol. Chem.,* 254, No. 16: 7621—7623. The other compounds respectively are believed to correspond to the 101—111, 101—112, 101—113, 101—114, and 101—115 sequence of LDH-$C_4$, contrary to Musick et al. In the sequence numbering convention used, there is no amino acid 104.

**Description of invention**

The present invention relates to novel antigenic linear peptides having chain length from 10 to 14 amino acids. These peptides all include the N-terminal sequence:

N-Arg-Met-Val-Ser-Gly-Gln-Thr-Arg-Leu

More specifically the invention generically comprises the following five peptide compounds.
(P-1)   N-Arg-Met-Val-Ser-Gly-Gln-Thr-Arg-Leu-C,
(P-2)   N-Arg-Met-Val-Ser-Gly-Gln-Thr-Arg-Leu-Asp-C,
(P-3)   N-Arg-Met-Val-Ser-Gly-Gln-Thr-Arg-Leu-Asp-Leu-C,
(P-4)   N-Arg-Met-Val-Ser-Gly-Gln-Thr-Arg-Leu-Asp-Leu-Leu-C,
(P-5)   N-Arg-Met-Val-Ser-Gly-Gln-Thr-Arg-Leu-Asp-Leu-Leu-Gln-C, and
(P-6)   N-Arg-Met-Val-Ser-Gly-Gln-Thr-Arg-Leu-Asp-Leu-Leu-Gln-Arg-C,
wherein the letter "N" designates the N-terminal amino acids, while the letter "C" designates the C-terminal amino acids. Gly represents glycine, and Arg, Met, Val, Ser, Gln, Thr, Leu, and Asp respectively represent the L-amino acid forms arginine, methionine, valine, serine, glutamine, threonine, leucine, and aspartic acid.

The peptide compounds of the present invention P-1, P-2, P-3, P-4, P-5, and P-6 can be synthesized from their constituent amino acids. For example, the synthesis can be carried out by the Merrifield solid phase method, as described in *J.A.C.S.* 85:2149—2154 (1963). This solid phase method for synthesizing sequences of amino acids is also described in Stewart and Young, *Solid Phase Peptide Synthesis* (W. H.

2

Freeman and Co., San Francisco, 1969), pages 1—4. In this procedure, the C-terminal amino acid, such as arginine for the P-1 compound of this invention is attached to chloromethylated polystyrenedivinylbenzene copolymer beads. Each subsequent amino acid, with suitable protecting group, is then added sequentially to the growing chain. For example, as described in the Merrifield article, the protective group may be a carbobenzoxy group. By the procedure of coupling, deprotection, and coupling of the next amino acid, the desired amino acid sequence and chain length can be produced. As a final step, the protective group is removed from the N-terminal amino acid such as from the N-terminal arginine, and then is cleaved from the resin, using a suitable reagent, such as trifluoroacetic acid and hydrogen bromide. Since this synthesis procedure is well known, it is not believed that it will be necessary to further describe it herein. The peptide of this invention can be prepared by this synthesis procedure for use in reducing the fertility of mammals.

To utilize the antigenic peptides of this invention (P-1 to P-6) in the form of fertility reducing vaccines, the peptide used is conjugated to a carrier molecule, which is preferably a protein which itself elicits an antigenic response and which can be safely administered. For example, the selected peptide can be coupled to tetanus toxoid for administration by intramuscular injection. For example, a mixture of 1µ Mole tetanus toxoid, 60 µ-Mole antigenic peptide, and 18 millimoles 1-ethyl-3-(3 dimethyl aminopropyl) carbodiimide hydrochloride reacted in water (pH 6) for 12 hours at room temperature and 24 hours at 4°C. gives a product containing 3.5 moles of peptide/mole of tetanus toxoid. Excess reactants can be removed by dialysis or gel filtration. See Pique et al, *Immunochemistry,* 15: 55—60 (1978). Alternatively, the peptide may be coupled using bisdiazotized benzidine (Bassiri et al, *Endocrinology,* 90: 722 (1972) or glutaraldehyde.

For intramuscular injection, the coupled peptide may be suspended in a sterile isotonic saline solution, or other conventional vehicle, and, if desired, an adjuvant may be included. A preferred use of such a vacine is for administration to human females. Antibodies will be formed, which will appear in the oviduct fluids and thereby achieve a significant reduction in fertility. For this purpose, the amount to be administered will range from about 1 to 10 milligrams (mg) of the antigenic peptide.

The peptide compounds of this invention and their method of preparation are further illustrated by the following examples.

Example I

Preparation of linear peptide

Arg-Met-Val-Ser-Gly-Gln-Thr-Arg-Leu-Asp-Leu-Leu-Gln-Arg

Synthesis of the above peptide (P-6) can be carried out employing solid phase techniques now well known in the art. In a preferred procedure amino protected arginine, representing the —COOH terminal group of the above peptide, is coupled to a conventional solid phase peptide synthesis resin such as chloromethyl polystyrene cross-linked with 1 to 2% divinyl benzene. The amino protecting group is then selectively removed utilizing a suitable reagent whose nature will depend on the protecting group used. In the preferred embodiment the t-butyloxycarbonyl (Boc) group is utilized for amino group protection and 40% trifluoroacetic acid in methylene chloride is the selective deprotecting agent.

After deprotection, the arginine resin is treated with protected glutamine, preferably N-Boc-Glutamine, and dicyclohexylcarbodiimide in a manner known per se as to form a peptide bond between the free amino group of the arginine residue and the carboxyl group of protected glutamine.

The cycle of deprotection and coupling with amino acid derivatives and dicyclohexylcarbodiimide is then repeated with the remaining amino acids in the sequence order of the above peptide. Some of the amino acids required side-chain blocking groups besides the alphaamino protection. Such amino acids and the blocking groups are as follows:

Met(MBzl), Ser (oBzl), Arg(Tos), Asp(oBzl), THR(oBzl)

Where oBzl is benzyl, Tos is guanidino-p-Toluene-sulfonyl and MBzl is methoxybenzyl.

Completion of the synthesis provided the following peptide coupled to the styrenedivinylbenzene copolymer resin:

TFA-Arg(Tos)-Met(mBzl)-Val-Ser(oBzl)-Gly-Gln-Thr(oBzl)-Arg(Tos)-Leu-Asp(oBzl)-Leu-Leu-Gln-Arg(Tos)-resin

Decoupling of the peptide from the resin is accomplished by treatment with liquid hydrogen fluoride with concomittant cleavage of all protecting groups to produce the desired peptide.

Attachment of N-Boc-Arginine(Tos) to chloromethyl resin was performed by the cesium salt method. A sample of chloromethyl resin (200 g.) containing 0.74 mmol chloride per gram is treated with the cesium salt of Boc-Arginine(Tos) resulting from the neutrallization of Boc-Arginine(Tos) with cesium carbonate. About 38.6 grams of Boc-Arginine(Tos) is dissolved in 80% methanol and 20% water and adjusted to pH 7.0 with about 29 grams of cesium carbonate. The resulting solution is dried on a rotary evaporator, then dried three more times after three additions of 100 milliliters of xylene. To the cesium salt of Boc-Arginine(Tos) was added 200 grams of chloromethyl resin as above and sufficient 1-Methyl-2-Pyrrolidinone to make about 1.90 liters total volume. The resulting mixture is stirred at 55°C. for 48 hours. The resin was then washed extensively with methanol, then water, then again with methanol. The resin was air dried, then dried under vacuum. After cleavage of Arginine from the resin with HF, amino acid analysis gave one peak corresponding to 0.48 mmol/gm.

A sample of the resin just described (6.0 g.) was submitted to the following synthesis schedule: (1) Wash with three 100 ml. portions of methylene chloride; (2) removal of the Boc group with 40% TFA in methylene chloride for a one minute wash and for a 20 minute reaction time; (3) wash with three 100 ml. portions of methylene chloride; (4) wash with two 100 ml. portions of isopropanol; (5) wash with three 100 ml. portions of methylene chloride; (6) a one minute wash and ten minutes neutrallization with 100 ml. portions of 10% triethylamine in methylene chloride; (7) wash with three portions of 100 ml. of methylene chloride; (8) add 2.5 equivalents (7.2 mmol) of Boc amino acid and 2.5 equivalents (7.2 mmol) of dicyclohexylcarbodiimide in methylene chloride and shake for 2 hours; (9) wash with three 100 ml. portions of methylene chloride; (10) wash with two 100 ml. portions of isopropanol; (11) wash with three 100 ml. portions of methylene chloride. The above cycle was repeated for the following N-protected amino acids:

| | |
|---|---|
| Boc-Gln | Boc-Thr(oBzl) |
| Boc-Leu | Boc-Gln |
| Boc-Leu | Boc-Gly |
| Boc-Asp(oBzl) | Boc-Ser(oBzl) |
| Boc-Leu | Boc-Val |
| Boc-Arg(Tos) | Boc-Met(mBzl) |
| | Boc-Arg(Tos) |

The protected peptide resin was submitted to deprotection to give the TFA salt of the protected peptide resin. The dried resin (5.88 g) was stirred in the presence of 6 ml. of anisole and 60 ml. of liquid HF at 0°C for 1 hour. The HF was removed by vacuum and the oily residue was washed with two 50 ml. portions of ethyl ether. The peptide was extracted from the resin by three 50 ml. portions of 1 molar acetic acid and the combined filtrates were lyophilized to give 2.27 grams of crude peptide. This was purified by 250 transfers in a counter-current distribution apparatus. The solvent system for the above fractionation was butanol:acetic acid:water at 4:1:5 ratios.

Somewhat purified fractions from the counter-current distribution apparatus were further purified by column chromatography on diethylaminoethylcellulose with a linear gradient of 0.01 to 0.5 molar ammonium bicarbonate to give 250 mg. of pure peptide.

Amino acid analysis of the pure peptide after acid hydrolysis gave: ammonia 1.76, Arg 2.91, Asp 0.99, Thr 1.00, Ser 0.99, Glu 2.091, Gly 1.01, Val 1.081, Met 0.97, Leu 3.04. This peptide gave a single spot with a Rf of 0.62 on cellulose thin layer chromatography with a solvent system of butanol:pyridine:acetic acid:water of 15:10:3:12, and an rF of 0.53 with these same solvents of 42:24:4:30.

Example II

The respective shorter chain sequence compounds of the invention are prepared by the method of Example I starting with the respective C-terminal amino acids; leucine for the 9-sequence compounds P-1, aspartic acid for the 10-sequence compounds P-2, leucine for the 11-sequence and 12-sequence compounds P-3 and P-4, and glutamine for the 13-sequence compound P-5, ending all sequences, as shown above, with the N-terminal arginine.

For example, the 9-sequence P-1 compound is prepared as follows:

Preparation of linear peptide
Arg-Met-Val-Ser-Gly-Gln-Thr-Arg-Leu

Synthesis of the above peptide can be carried out employing solid phase techniques now well known in the art. In a preferred procedure amino protected leucine, representing the —COOH terminal group of the above peptide, is coupled to a conventional solid phase peptide synthesis resin such as chloromethyl polystyrene cross-linked with 1 to 2% divinyl benzene. The amino protecting group is then selectively removed utilizing a suitable reagent whose nature will depend on the protecting group used. In the preferred embodiment the t-butyloxycarbonyl (Boc) group is utilized for amino group protection and 40% trifluoroacetic acid in methylene chloride is the selective deprotecting agent.

After deprotection, the leucine resin is treated with protected arginine, preferably N-Boc-N$^b$-Tosyl-arginine, and dicyclohexylcarbodiimide in a manner known per se as to form a peptide bond between the free amino group of the leucine residue and the carboxyl group of protected arginine.

The cycle of deprotection and coupling with amino acid derivatives and dicyclohexylcarbodiimide is then repeated with the remaining amino acids in the sequence order of the above peptide. Some of the

4

# 0 101 677

amino acids required side-chain blocking groups besides the alphaamino protection. Such amino acids and the blocking groups are as follows:

Met(MBzl), Ser(oBzl), Arg(Tos), Thr(oBzl)

Where oBzl is benzyl, Tos is guanidino-p-Toluenesulfonyl and MBzl is methoxybenzyl.

Completion of the synthesis provided the following peptide coupled to the styrenedivinylbenzene copolymer resin:

TFA-Arg(Tos)-Met(mBzl)-Val-Ser(oBzl)-Gly-Gln-Thr(oBzl)-Arg(Tos)-Leu-resin

Decoupling of the peptide from the resin is accomplished by treatment with liquid hydrogen fluoride with concomittant cleavage of all protecting groups to produce the desired peptide.

Attachment of N-Boc-leucine to chloromethyl resin was performed by the cesium salt method. A sample of chloromethyl resin (200 g.) containing 0.74 mmol chloride per gram is treated with the cesium salt of Boc-leucine resulting from the neutralization of Boc-leucine with cesium carbonate. About 38.6 grams of Boc-leucine is dissolved in 80% methanol and 20% water and adjusted to pH 7.0 with about 29 grams of cesium carbonate. The resulting solution is dried on a rotary evaporator, then dried three more times after three additions of 100 milliliters of xylene. To the cesium salt of Boc-leucine was added 200 grams of chloromethyl resin as above and sufficient 1-Methyl-2-Pyrrolidinone to make about 1.90 liters total volume. The resulting mixture is stirred at 55°C. for 48 hours. The resin was then washed extensively with methanol, then water, then again with methanol. The resin was air dried, then dried under vacuum. After cleavage of leucine from the resin with HF, amino acid analysis gave one peak corresponding to 0.48 mmol/gm.

A sample of the resin just described (6.0 g.) was submitted to the following synthesis schedule: (1) wash with three 100 ml. portions of methylene chloride; (2) removal of the Boc group with 40% TFA in methylene chloride for a one minute wash and for a 20 minute reaction time; (3) wash with three 100 ml. portions of methylene chloride; (4) wash with two 100 ml. portions of isopropanol; (5) wash with three 100 ml. portions of methylene chloride; (6) a one minute wash and ten minutes neutrallization with 100 ml. portions of 10% triethylamine in methylene chloride; (7) wash with three portions of 100 ml. of methylene chloride; (8) add 2.5 equivalents (7.2 mmol) of Boc amino acid and 2.5 equivalents (7.2 mmol) of dicyclohexylcarbodiimide in methylene chloride and shake for 2 hours; (9) wash with three 100 ml. portions of methylene chloride; (10) wash with two 100 ml. portions of isopropanol; (11) wash with three 100 ml. portions of methylene chloride. The above cycle was repeated for the following N-protected amino acids:

Boc-Arg(Tos)

Boc-Thr(oBzl)

Boc-Gln

Boc-Gly

Boc-Ser(oBzl)

Boc-Val

Boc-Met(mBzl)

Boc-Arg(Tos)

The protected peptide resin was submitted to deprotection to give the TFA salt of the protected peptide resin. The dried resin (5.88 g) was stirred in the presence of 6 ml. of anisole and 60 ml. of liquid HF at 0°C for 1 hour. The HF was removed by vacuum and the oily residue was washed with two 50 ml. portions of ethyl ether. The peptide was extracted from the resin by three 50 ml. portions of 1 molar acetic acid and the combined filtrates were lyophilized to give 2.27 grams of crude peptide. This was purified by 250 transfers in a counter-current distribution apparatus. The solvent system for the above fractionation was butanol:acetic acid:water at 4:1:5 ratios.

Somewhat purified fractions from the counter-current distribution apparatus were further purified by column chromatography on diethylaminoethylcellulose with a linear gradient of 0.01 to 0.5 molar ammonium bicarbonate to give 250 mg. of pure peptide.

**Claims for the Contracting States: DE, FR, GB, LU, NL, SE**

1. The antigenic peptide compounds having chain lengths of from 9 to 14 amino acids arranged in a sequence from N-terminal to C-terminal amino acids which include the antigenic sequence

5

**0 101 677**

Arg-Met-Val-Ser-Gly-Gln-Thr-Arg-Leu,

said compounds being selected from the class of compounds consisting of:
(a) Arg-Met-Val-Ser-Gly-Gln-Thr-Arg-Leu,
(b) Arg-Met-Val-Ser-Gly-Gln-Thr-Arg-Leu-Asp,
(c) Arg-Met-Val-Ser-Gly-Gln-Thr-Arg-Leu-Asp-Leu,
(d) Arg-Met-Val-Ser-Gly-Gln-Thr-Arg-Leu-Asp-Leu-Leu,
(e) Arg-Met-Val-Ser-Gly-Gln-Thr-Arg-Leu-Asp-Leu-Leu-Gln, and
(f) Arg-Met-Val-Ser-Gly-Gln-Thr-Arg-Leu-Asp-Leu-Leu-Gln-Arg,
wherein Gly represents glycine, and Arg, Met, Val, Ser, Gln, Thr, Leu, Asp, respectively represent the L-amino acid forms of arginine, methionine, valine, serine, glutamine, threonine, leucine, and aspartic acid.

2. The antigenic peptide of claim 1 having the amino acid sequence (a).
3. The antigenic peptide of claim 1 having the amino acid sequence (b).
4. The antigenic peptide of claim 1 having the amino acid sequence (c).
5. The antigenic peptide of claim 1 having the amino acid sequence (d).
6. The antigenic peptide of claim 1 having the amino acid sequence (e).
7. The antigenic peptide of claim 1 having amino acid sequence (f).

**Claims for the Contracting State: AT**

1. A method for preparing antigenic peptide compounds having chain lengths of from 9 to 14 amino acids arranged in a sequence from N-terminal to C-terminal amino acids which include the antigenic sequence

Arg-Met-Val-Ser-Gly-Gln-Thr-Arg-Leu,

said compounds being selected from the class of compounds consisting of:
(a) Arg-Met-Val-Ser-Gly-Gln-Thr-Arg-Leu,
(b) ·Arg-Met-Val-Ser-Gly-Gln-Thr-Arg-Leu-Asp,
(c) Arg-Met-Val-Ser-Gly-Gln-Thr-Arg-Leu-Asp-Leu,
(d) Arg-Met-Val-Ser-Gly-Gln-Thr-Arg-Leu-Asp-Leu-Leu,
(e) Arg-Met-Val-Ser-Gly-Gln-Thr-Arg-Leu-Asp-Leu-Leu-Gln, and
(f) Arg-Met-Val-Ser-Gly-Gln-Thr-Arg-Leu-Asp-Leu-Leu-Gln-Arg,
wherein Gly represents glycine, and Arg, Met, Val, Ser, Gln, Thr, Leu, Asp, respectively represent the L-amino acid forms of arginine, methionine, valine, serine, glutamine, threonine, leucine, and aspartic acid.

2. A method of claim 1 having the amino acid sequence (a).
3. A method of claim 1 having the amino acid sequence (b).
4. A method of claim 1 having the amino acid sequence (c).
5. A method of claim 1 having the amino acid sequence (d).
6. A method of claim 1 having the amino acid sequence (e).
7. A method of claim 1 having the amino acid sequence (f).

**Patentansprüche für die Vertragsstaaten: DE, FR, GB, LU, NL, SE**

1. Antigene Peptidverbindungen, die eine Kettenlänge von 9 bis 14 Aminosäuren aufweisen, die in einer Sequenz von N-terminalen zu C-terminalen Aminosäuren angeordnet sind, die die antigene Sequenz

Arg-Met-Val-Ser-Gly-Gln-Thr-Arg-Leu

enthält, wobei die Verbindungen ausgewählt werden aus einer Klasse von Verbindungen, die die folgenden Sequenzen enthält:
(a) Arg-Met-Val-Ser-Gly-Gln-Thr-Arg-Leu,
(b) Arg-Met-Val-Ser-Gly-Gln-Thr-Arg-Leu-Asp,
(c) Arg-Met-Val-Ser-Gly-Gln-Thr-Arg-Leu-Asp-Leu,
(d) Arg-Met-Val-Ser-Gly-Gln-Thr-Arg-Leu-Asp-Leu-Leu,
(e) Arg-Met-Val-Ser-Gly-Gln-Thr-Arg-Leu-Asp-Leu-Leu-Gln und
(f) Arg-Met-Val-Ser-Gly-Gln-Thr-Arg-Leu-Asp-Leu-Leu-Gln-Arg,
wobei Gly die Aminosäure Glycin bedeutet und Arg, Met, Val, Ser, Gln, Thr, Leu und Asp die L-Aminosäureformen von jeweils Arginin, Methionin, Valin, Serin, Glutamin, Threonin, Leucin und Asparaginsäure bedeuten.

2. Ein antigenes Peptid nach Anspruch 1, das die Aminosäuresequenz (a) aufweist.
3. Ein antigenes Peptid nach Anspruch 1, das die Aminosäuresequenz (b) aufweist.
4. Ein antigenes Peptid nach Anspruch 1, das die Aminosäuresequenz (c) aufweist.
5. Ein antigenes Peptid nach Anspruch 1, das die Aminosäuresequenz (d) aufweist.
6. Ein antigenes Peptid nach Anspruch 1, das die Aminosäuresequenz (e) aufweist.
7. Ein antigenes Peptid nach Anspruch 1, das die Aminosäuresequenz (f) aufweist.

6

**0 101 677**

**Patentansprüche für den Vertragsstaat: AT**

1. Verfahren zum Herstellen einer antigenen Peptidverbindung, die eine Kettenlänge von 9 bis 14 Aminosäuren aufweist, die in einer Sequenz von N-terminalen zu C-terminalen Aminosäuren angeordnet sind, die die antigene Sequenz

Arg-Met-Val-Ser-Gly-Gln-Thr-Arg-Leu

enthält, wobei die Verbindungen ausgewählt werden aus einer Klasse von Verbindungen, die die folgenden Sequenzen enthält:
- (a) Arg-Met-Val-Ser-Gly-Gln-Thr-Arg-Leu,
- (b) Arg-Met-Val-Ser-Gly-Gln-Thr-Arg-Leu-Asp,
- (c) Arg-Met-Val-Ser-Gly-Gln-Thr-Arg-Leu-Asp-Leu,
- (d) Arg-Met-Val-Ser-Gly-Gln-Thr-Arg-Leu-Asp-Leu-Leu,
- (e) Arg-Met-Val-Ser-Gly-Gln-Thr-Arg-Leu-Asp-Leu-Leu-Gln, und
- (f) Arg-Met-Val-Ser-Gly-Gln-Thr-Arg-Leu-Asp-Leu-Leu-Gln-Arg,

wobei Gly die Aminosäure Glycin bedeutet, und Arg, Met, Val, Ser, Gln, Thr, Leu and Asp die L-Aminosäureformen von jeweils Arginin, Methionin, Valin, Serin, Glutamin, Threonin, Leucin und Asparaginsäure bedeuten.

2. Verfahren nach Anspruch 1, wobei die Aminosäuresequenz die Sequenz (a) ist.

3. Verfahren nach Anspruch 1, wobei die Aminosäuresequenz die Sequenz (b) ist.

4. Verfahren nach Anspruch 1, wobei die Aminosäuresequenz die Sequenz (c) ist.

5. Verfahren nach Anspruch 1, wobei die Aminosäuresequenz die Sequenz (d) ist.

6. Verfahren nach Anspruch 1, wobei die Aminosäuresequenz die Sequenz (e) ist.

7. Verfahren nach Anspruch 1, wobei die Aminosäuresequenz die Sequenz (f) ist.

**Revendications Pour les états Contractants: DE, FR, GB, LU, NL, SE**

1. Composés peptidiques antigéniques ayant des longueurs de chaîne de 9 à 14 acides aminés disposés selon une séquence allant des acides aminés à N terminal aux acides aminés à C terminal, qui comprennent la séquence antigénique

Arg-Met-Val-Ser-Gly-Gln-Thr-Arg-Leu,

lesdits composés étant sélectionnés dans la classe des composés constitués par:
- (a) Arg-Met-Val-Ser-Gly-Gln-Thr-Arg-Leu,
- (b) Arg-Met-Val-Ser-Gly-Gln-Thr-Arg-Leu-Asp,
- (c) Arg-Met-Val-Ser-Gly-Gln-Thr-Arg-Leu-Asp-Leu,
- (d) Arg-Met-Val-Ser-Gly-Gln-Thr-Arg-Leu-Asp-Leu-Leu,
- (e) Arg-Met-Val-Ser-Gly-Gln-Thr-Arg-Leu-Asp-Leu-Leu-Gln, et
- (f) Arg-Met-Val-Ser-Gly-Gln-Thr-Arg-Leu-Asp-Leu-Leu-Gln-Arg,

où Gly représente la glycine et Arg, Met, Val, Ser, Gln, Thr, Leu, Asp représentent respectivement les formes L des acides aminés arginine, méthionine, valine, sérine, glutamine, thréonine, leucine et acide aspartique.

2. Peptide antigénique de la revendication 1, ayant la séquence d'acides aminés (a).

3. Peptide antigénique de la revendication 1, ayant la séquence d'acides aminés (b).

4. Peptide antigénique de la revendication 1, ayant la séquence d'acides aminés (c).

5. Peptide antigénique de la revendication 1, ayant la séquence d'acides aminés (d).

6. Peptide antigénique de la revendication 1, ayant la séquence d'acides aminés (e).

7. Peptide antigénique de la revendication 1, ayant la séquence d'acides aminés (f).

**Revendications pour l'Etat Contractant: AT**

1. Procédé de préparation de composés peptidiques antigéniques ayant des longueurs de chaîne de 9 à 14 acides aminés disposés selon une séquence allant des acides aminés à N-terminal aux acides aminés à C-terminal, qui comprennent la séquence antigénique

Arg-Met-Val-Ser-Gly-Gln-Thr-Arg-Leu,

lesdits composés étant sélectionnés dans la classe des composés constitués par:
- (a) Arg-Met-Val-Ser-Gly-Gln-Thr-Arg-Leu,
- (b) Arg-Met-Val-Ser-Gly-Gln-Thr-Arg-Leu-Asp,
- (c) Arg-Met-Val-Ser-Gly-Gln-Thr-Arg-Leu-Asp-Leu,
- (d) Arg-Met-Val-Ser-Gly-Gln-Thr-Arg-Leu-Asp-Leu-Leu,
- (e) Arg-Met-Val-Ser-Gly-Gln-Thr-Arg-Leu-Asp-Leu-Leu-Gln, et
- (f) Arg-Met-Val-Ser-Gly-Gln-Thr-Arg-Leu-Asp-Leu-Leu-Gln-Arg,

7

où Gly représente la glycine, et Arg, Met, Val, Ser, Gln, Thr, Leu, Asp, représentent respectivement les. formes L des acides aminés arginine, méthionine, valine, sérine, glutamine, thréonine, leucine et acide aspartique.

2. Procédé de la revendication 1 ayant la séquence d'acides aminés (a).
3. Procédé de la revendication 1 ayant la séquence d'acides aminés (b).
4. Procédé de la revendication 1 ayant la séquence d'acides aminés (c).
5. Procédé de la revendication 1 ayant la séquence d'acides aminés (d).
6. Procédé de la revendication 1 ayant la séquence d'acides aminés (e).
7. Procédé de la revendication 1 ayant la séquence d'acides aminés (f).